Europäisches Patentamt

European Patent Office

Office européen des brevets

(19)

(11) Veröffentlichungsnummer: **0 112 979**
**B1**

(12) **EUROPÄISCHE PATENTSCHRIFT**

(45) Veröffentlichungstag der Patentschrift:
04.02.87

(51) Int. Cl.⁴: **A 61 M 16/00**

(21) Anmeldenummer: **83109830.6**

(22) Anmeldetag: **01.10.83**

(54) Atemhilfegerät.

(30) Priorität: 05.11.82 DE 3240897

(43) Veröffentlichungstag der Anmeldung:
11.07.84 Patentblatt 84/28

(45) Bekanntmachung des Hinweises auf die Patenterteilung:
04.02.87 Patentblatt 87/6

(84) Benannte Vertragsstaaten:
CH DE FR GB LI SE

(56) Entgegenhaltungen:
WO-A-81/00212
DE-A-1 912 572
DE-A-2 927 839
GB-A-2 012 172
GB-A-2 054 387
US-A-2 584 450
US-A-4 082 093

(73) Patentinhaber: Obermayer, Anton, Dipl.- Ing.,
Parkstrasse 4, D-7954 Bad Wurzach (DE)

(72) Erfinder: Obermayer, Anton, Dipl.- Ing.,
Parkstrasse 4, D-7954 Bad Wurzach (DE)

(74) Vertreter: Patentanwälte Dipl.- Ing. E. Eisele Dr.-
Ing. H. Otten, Seestrasse 42, D-7980 Ravensburg
(DE)

LIBER, STOCKHOLM 1987

EP 0 112 979 B1

## Beschreibung

Die Erfindung betrifft ein Atemhilfegerät nach dem Oberbegriff des Anspruchs 1.

Die in den letzten Jahren wesentlich erweiterte Indikationsstellung in Bezug auf die Schwere der operativen Eingriffe und das Patientenalter, erfordert eine dementsprechende Entwicklung der Beatmungstechnik. Aus der anfänglichen, einfachen Unterstützung der Spontanatmung während längerer Narkosen entwickelte sich eine sogenannte Atemtherapie, die sich zahlreicher Beatmungsformen und -geräten bedient.

Der schwierigste Abschnitt jeder Respirator-Therapie umfaßt die Entwöhnung und Wiederherstellung der Spontanatmung des Patienten nach länger dauernder Beatmung auf der Intensivstation. Als letzter Schritt der Übergangsphase findet heute die sogenannte CPAP-Atmung Anwendung. Hierbei atmet der Patient spontan an einem Beatmungsgerät mit einem um 5 - 10 mbar erhöhtem Gesamtdruckniveau. Durch diese Maßnahme wird die Lunge dauernd in einem geblähten und damit volumenvergrößerten Zustand gehalten, so daß eine verbesserte Sauerstoffzuführung erzielt und die ggf. hochfrequente Hechelatmung unterdrückt werden kann.

Zur Durchführung der CPAP-Atmung, d. h. einer Atmung unter einem kontinuierlich positiven Atemwegdruck, werden entweder schwere Beatmungsmaschinen, die über diese Beatmungsform verfügen oder aber spezielle CPAP-Geräte eingesetzt. Die Erzeugung und Sicherstellung des andauernden positiven Druckniveaus im gesamten Respirationstrakt erfolgt bei den bekannten Geräten nach zwei Prinzipien, nämlich dem High-Flow-CPAP oder dem Demand-CPAP.

Beim High-Flow-CPAP wird dem Patienten ein ständiger Gasstrom angeboten. Der gewünschte Überdruck im Respirationstrakt wird durch ein Überdruckventil bestimmt, z. B. durch die Eintauchtiefe eines Exspirationsrohres in einem Wasserschloß. High-Flow-GPAP-Geräte sind zwar preisgünstig, sie weisen jedoch eine Reihe konstruktiv bedingter Nachteile auf. Hier sind insbesondere die großen exspiratorischen Atemwegwiderstände, d. h. Widerstände beim Ausatmen des Patienten zu nennen, mit den entsprechenden negativen Über- und Unterschwingweiten um dasgewünschte, am Gerät eingestellte Druckniveau (Peep-Niveau).

Durch das High-Flow-CPAP-Gerät fließt demnach ein ständiger Gasstrom unter einem positiven Druckniveau von 5 - 10 mbar.

Dem patienten steht deshalb ein ständiger Gasstrom über ein patienten-Anschluß-T-Stück zur Verfügung. Da der Gasverbrauch auf die höchstmögliche Entnahme des Patienten eingestellt werden muß, ergibt sich ein hoher Gasverbrauch dadurch, daß in der Exspirationsphase das Gas ungenutzt aus dem Überströmventil (Peep-Ventil) ausströmt. Die hohen Atemwegwiderstände in der Exspirationsphase ergeben sich dadurch, daß zusätzlich zum ständigen Gasfluß die ausgeatmete Luft hinzukommt, so daß sich eine Druckerhöhung im exspiratorischem Atemzweig einstellt. Infolge des beim Einatmen auftretenden Unterdrucks im inspiratorischen- infolge des Gasstaus auftretende Überdrucks im exspiratorischen Zweig stellen sich bei den bekannten High-Flow-CPAP-Gerät große Unter- und überschwingweiten der Atemdruckkurve um das eingestellte Peep-Niveau ein. Dies stellt jedoch eine zusätzliche Belastung des patienten dar.

Da der exspiratorische Zweig in der Ausatmungsphase sowohl den stündigen Gasstrom als auch die ausgeatmete Luft aufnimmt, ist eine Messung des Atemvolumens nicht oder nur unzureichend über eine Pifferenzmessung möglich, wobei Gasverluste im System zu berücksichtigen wären. Die Messung des Atemvolumens ist jedoch für die Feststellung des Sauerstoffgehalts im Blut von großer Bedeutung.

Ein bekanntes Gerät nach dem High-Flow-CPAP-Prinzip ist in der Durckschrift GB-A-2 012 172 beschrieben. Bei diesem bekannten Gerät erfolgt die Erzeugung des Peep-Druckes über eine separate Drucksteuerung mit separater Steuergasleitung und separatem Steuergasverbrauch. Ein im inspiratorischen Zweig vorgesehenes Druckbegrenzungsventil mit separater Ansteuerung dient zur maximalen Druckbegrenzung in diesem Zweig und ist unabhängig vom Peep-Druck. Während der Exspirationsphase fließt der Gasstrom aus dem Inspirationszweig parallel zum Exspirationsgas des Patienten über ein Exspirationsventil ins Freie.

Beim Demand-CPAP, welches hauptsächlich in schweren Beatmungsgeräten zu finden ist, wird der vom Patienten gewünschte inspiratorische Gasstrom über einen Drucksensor und ein regelbares Inspirationsventil bedarfsgerecht angeboten. Hierdurch werden die Nachteile des High-Flow-CPAP zwar teilweise vermieden, da nur das jeweils gewünschte Atemzugvolumen geliefert und die vorhandenen Monitoringsysteme benützt werden können. Nachteilig wirken sich jedoch die erforderlichen Messund Schaltzeiten des Drucksensors und des Inspirationsventils aus, d. h., der Patient bekommt das Atemgas zunächst nicht oder in zu geringer Menge und später schubweise oder in zu großen Mengen geliefert.

Die zusätzlich erforderlichen Exspirationsventile weisen ferner erhebliche Strömungswiderstände auf oder begrenzen infolge ungenügender Öffnungswinkel die Strömungsgeschwindigkeit der Exspirationsluft. Während der Exspirationsphase wird der Atemwegdruck von einem Druckwandler in der Exspirationsseite gemessen. Das Signal vom Wandler wird mit dem vorgegebenen peep-Niveau verglichen. Eine Differenz zwischen tatsächlichem und eingestelltem Wert resultiert in einem Signal an das Exspirationsventil. Dieses Signal korrigiert das Exspirationsventil ständig, so daß das gewünschte Peep-Niveau erhalten wird.

Da das Exspirationsventil während der Inspirations- und Pausenphase geschlossen bleibt und danach geregelt öffnet, ergeben sich auch hier Zeitverzögerungen und damit verbundene Druckschwankungen, die sich auf die gewünschte überschwingweite des Druckniveaus ebenfalls negativ auswirken.

Ein bekanntes Gerät welches nach dem Demand-Prinzip arbeitet ist in der Druckschrift GB-A-2 054 387 wiedergegeben. Hier ist eine Atemgaskammer vorgesehen, die mittels eines über eine Steuereinheit geregelten Ejektor mit Gas derart beaufschlagt wird, daß sich ein Peep-Niveau im Inspirations- und im Exspirationszweig einstellt. Die Atemgaskammer wird während jeder Einatmungsphase vollständig geleert, so daß dem Patienten ein vorbestimmtes konstantes Atemgasvolumen zugeführt wird. Durch die aufwendige Regelung über die Steuereinheit lassen sich die vorgenannten Nachteile nicht vermeiden.

Es ist deshalb grundsätzlich festzustellen, daß weder das High-Flow- noch das Demand-System den Erfordernissen einer idealen CPAP-Atmung d. h. einer Atmung mit möglichst kleinen Unter- und überschwingweiten um das eingestellte Druckniveau gerecht wird.

Da das Demand-CPAP-System nur in schweren und damit teueren Beatmungsmaschinen zu finden ist, werden die Patienten normalerweise mit dem High-Flow-CPAP-System behandelt. Die auch hiermit verbundenen Nachteile wurden bereits angegeben.

Der Erfindung liegt die Aufgabe zugrunde, ein kostengünstiges Atemhilfegerät der gattungsgemäßen Art zu schaffen, welches die Vorteile der nach dem High-Flow- und dem Demand-Prinzip arbeitenden Geräte verbindet, bei welchem insbesondere in der Exspirationsphase ein möglichst geringer Atemwegwiderstand vorhanden ist, welches eine verzögerungsfreie und damit "weiche" Kopplung zwischen Patient und Gerät in der Inspirationsphase ermöglicht und bei welchem geringe Druckschwankungen in definierter Größe um das benötigte Druckniveau und damit eine geringe Patientenbelastung vorhanden ist, und bei welchem insbesondere eine Messung ausschließlich des exspirierten Gases ermöglicht wird.

Diese Aufgabe wird erfindungsgemäß durch die kennzeichnenden Merkmale des Anspruchs 1 gelöst.

Die Erfindung vereint demnach die Vorteile beider bekannten Systeme, ohne die Hauptnachteile in Kauf nehmen zu müssen. Die konstruktive Ausführungsform des erfindungsgemäßen Gerätes stellt ebenfalls eine Kombination der bekannten Systeme jedoch mit dem wesentlichen Unterschied dar, daß das Ventil zur Einstellung des Druckniveaus (Peep-Ventil) nicht mehr im Expirationszweig wie beim High-Flow-CPAP-Bystem sondern im Inspirationszweig angeordnet ist, und daß das erfindungsgemäße Exspirationsventil nur noch eine Auf/Zu Funktion hat.

Ähnlich wie beim High-Flow-CPAP-System erfolgt demnach eine "weiche Kopplung" zwischen Atemhilfegerät und Patient durch einen ständigen Gasstrom. Schaltverzögerungen, die beim Triggern der schweren Beatmungsmaschine mit Hilfe des beim Atmungsvorgang entstehenden Unterdrucks entstehen, treten nicht auf, so daß die Unterschwingweiten des Druckniveaus klein gehalten werden können. Da sich das Peep-Ventil erfindungsgemäß im Inspirationszwelg des Atemhilfegeräts befindet, strömt im Eüpirationszweig nur noch die ausgeatmete Luft des Fatienten, wodurch der Atemwegwiderstand erheblich reduziert wird und eine genaue Messung des Atemvolumens möglich wird, zur Feststellung der Spontanatemfähigkeit, der Beatmungsparameter, wie Atemfrequenz, $CO_2$- und $O_2$-Austausch (Monitoring). Außerdem wird nunmehr eine automatische Überwachung ermöglicht. Die Messung im Exspirationszweig ist auch insofern wichtig, da derartige Geräte eine gewisse Leckrate aufweisen, d. h. bei Messung des Durchgangsvolumens im Inspirationszweig ist nicht erkennbar, inwieweit hier noch Leckverluste auftreten werden. Das am Ende des Exspirationszweigs angeordnete Exspirationsventil hat lediglich eine Auf/Zu Funktion, d. h. über dem eingestellten Druckniveau kann die ausgeatmete Luft das Ventil passieren.

Durch die in abhängigen Patentansprüchen aufgeführten Maßnahmen ist eine vorteilhafte Weiterbildung und Verbesserung des im unabhängigen Patentanspruch angegebenen Atemhilfegeräts möglich.

Gemäß der vorteilhaften Ausbildung nach Anspruch 2 wird als Anschluß zum Patienten ein Anschluß-T-Stück verwendet.

Die Ausbildung der Erfindung nach Anspruch 3 sieht die Verwendung eines Glimmerventils vor. Dieses Ventil ist derart eingestellt, daß es sich beim Aufbau eines über dem Peep-Niveau liegenden Druckes im exspiratorischen Zweig schließt, hervorgerufen durch den Ausatmungsvorgang des Patienten. Das Einwegventil wird möglichst nahe zum Patientenanschluß gelegt, um möglichst den gesamten inspiratorischen Zweig während der Exspirationsphase abzuschließen, bzw. den Druckverlust und damit den Atemwegwiderstand im inspiratorischen Zweig so gering als möglich zu halten.

Gemäß dem Anspruch 4 hat sich als einfaches Peep-Ventil ein Standrohr mit Wasserschloß bewährt, dessen Druckniveau von 5 - 10 mbar einstellbar ist. In bestimmten Anwendungsfällen sind jedoch auch niedrigere oder höhere Drücke notwendig.

Gemäß dem weiteren Anspruch 5 ist eine vorteilhafte Ausbildung des Exspirationsventils vorgesehen, wobei gewährleistet sein muß, daß beim Ausatmen (Exspirationsphase) das Mindestdruckniveau erhalten bleiben muß, d. h. das Ausatmen gegen den Druck des Peep-Niveaus erfolgt. Dies kann ein durch ein vom inspiratorischen Zweig gesteuertes Druckventil sein. Eine aufwendigere und Wieder mit Zeitverzögerung arbeitende Methode stellt ein regelbares Exspirationsventil dar.

Um den Nachteil des hohen Gasverbrauches bei den bekannten High-Flow-CPAP-Geräten zu vermeiden, arbeitet die Erfindung in einem bevorzugten Ausführungsbeispiel nach einem Low-Flow-CPAP-Prinzip, d. h. als ständiger Gasfluß wird eine niedrigere Gasmenge gewählt. Sofern der Patient einen höheren inspiratorischen Gasbedarf hat, sieht die Erfindung einen zusätzlichen Druckkonstanthalter vor, dessen Gas bei Bedarf dem inspiratorischen Zweig zugegeben wird. Um den Druckkonstanthalter zu füllen, ist ein Hochdruckgasmischer vorgesehen, der den Faltenbalg des Druckkonstanthalters bereits während der Entleerung und der exspiratorischen Phase mit Gas wieder füllt. Eine Drosseleinrichtung senkt den Druck des Hochdruckgasmischers auf den Druck im inspiratorischen Zweig, d. h. auf Peep-Niveau. Diese Drosseleinrichtung kann als Durchflußmesser mit Einstellventil zur Druckreduzierung ausgebildet sein. Der Druck im Druckkonstanthalter kann geringfügig über dem Druck des Peep-Niveaus liegen (Ansprüche 10, 11).

Durch vorstehende Maßnahme ist eine erhebliche Gasverbrauchsreduzierung und damit Kostenreduzierung möglich, wobei auf den Vorteil der "weichen Kopplung" zwischen Gerät und Patient nicht verzichtet werden braucht.

Durch die erfindungsgemäßen Maßnahmen werden demnach die Vorteile der bekannten Geräte vereinigt, ohne daß deren Nachteile - außer einem geringen Gasverbrauch - in Kauf zu nehmen sind. Das erfindungsgemäße Gerät ist derart einfach konstruiert, daß es preismäßig mit dem High-Flow-CPAP-Gerät konkurieren kann, jedoch nicht dessen Nachteile, insbesondere im exspiratorischen Zweig aufweist. Das Gerät liefert unter dem jeweils eingestellten Atemwegdruck das vom Patienten benötigte Volumen. Hat der Patient seinen Bedarf gedeckt, so wird keine aufwendige Regelung benötigt, da das Einwegventil den inspiratorischen Zweig abtrennt und die Exspiration erfolgen kann. Während dessen entweicht das laufend gelieferte Gas über das Peep-Ventil, ohne daß eine Regelung oder Steuerung eines Inspirationsventils notwendig ist. Der Patient hat immer die Möglichkeit, das Atmungsvolumen beliebig durch Eigenatmung zu vergrößern. Dabei ist der Patient durch geringe Unter- und Überschwingweiten um das eingestellte Peep-Niveau minimalst belastet. In Abwandlung eignet sich die Erfindung jedoch auch ohne die Einstellung eines ständigen positiven Atemwegdrucks (CPAP), bzw. einem Atemwegdruck der nahezu als Normaldruck zu bezeichnen wäre. Dies kann durch Einstellung am Peep-Ventil selbst geschehen.

In Ausgestaltung der Erfindung kann diese auch auf vorteilhafte Weise als Beatmungsgerät ("Plow-Zerhacker") verwendet werden, indem ein über eine Zeit-Takt-Schaltung geregeltes Auf-/Zu-Ventil dem Peep-Ventil nachgeschaltet wird. Durch Schließen bzw. Regelung des Ventils kann eine zusätzliche Beatmung in den verschiedensten Formen erfolgen (Unteransprüche 8, 9).

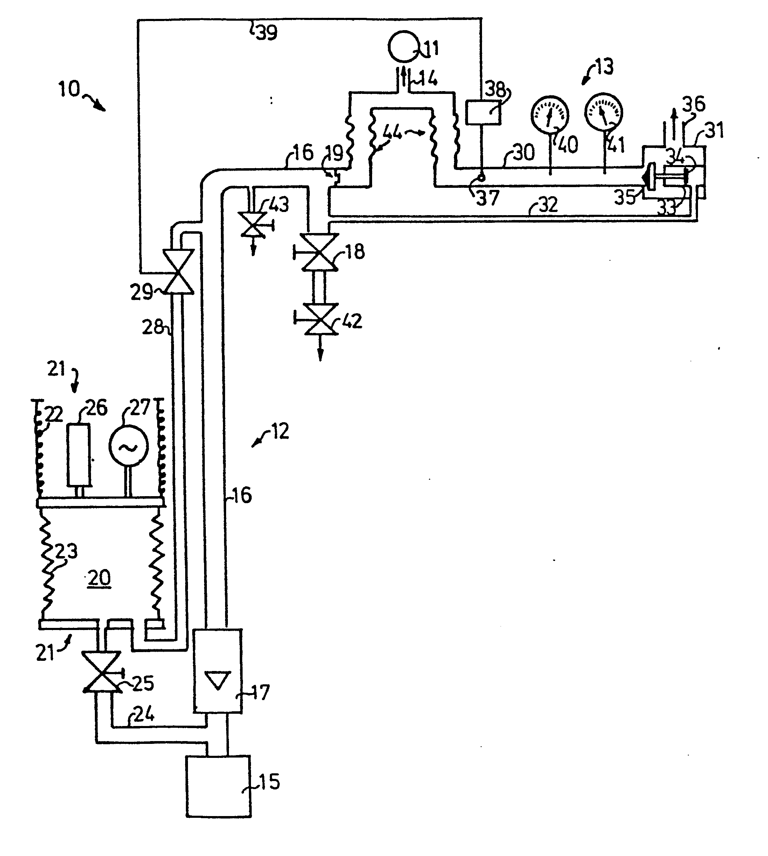

Ein zusätzliches Überdruck-Sicherheitsventil dient der Sicherheit des Patienten bei Gerät- bzw. Ventilstörungen. Ein vorteilhaftes Ausführungsbeispiel ist in der Figur dargestellt und nachfolgend näher beschrieben.

Die Figur zeigt eine schematische Darstellung des Aufbaus des erfindungsgemäßen Atemhilfegeräts.

Das erfindungsgemäße Atemhilfegerät 10 zum Anschluß an einen Patienten 11 besteht aus einem inspiratorischen Zweig 12 und einem exspiratorischen Zweig 13. Zwischen diesen beiden Zweigen ist ein Patienten-Anschluß-T-Stück 14 vorgesehen, im allgemeinen über einen Anschluß mittels eines Patienten-Schlauchsystems 44.

Von einem Hochdruckgasmischer 15 wird das jeweils gewünschte Gasgemisch - normalerweise mit Sauerstoff angereicherte Raumluft - in den Inspirationskanal 16 des Inspirationszweigs 12 geliefert. Das vom Hochdruckgasmischer mit ca. 5 bar gelieferte Gas wird über ein Rotameter 17 mit Drosselventil auf einen niedrigeren Druck abgesenkt. Durch ein sogenanntes Peep-Ventil 1ß im Inspirationszweig 12 wird ein Druck eingestellt, der etwa 5 - 10 mbar über dem Normaldruck liegt.

Im Inspirationszweig 12 ist weiterhin im Bereich des Inspirationsausganges ein Einwegventil 19 vorgesehen.

Der Inspirationszweig 12 besteht aus einem Direktversorgungsteil mit dem Inspirationskanal 16 und einem Speicher 20. Der Speicher 20 dient als Druckkonstanthalter 21 und weist einen unter dem Federdruck stehenden Faltenbalg 23 Dieser Balg 23 wird über die Leitung 24 und einem Füllstandsregelventil 25 von dem Hochdruckgasmischer 15 gefüllt. In dem dargestellten Ausführungsbeispiel sind zwei Antriebsvarianten für den Balg 23 dargestellt, mit deren Hilfe der Balg zusammengepreßt und der Druckkonstanthalter entleert werden kann. Zum einen ist hierzu eine hydraulische oder pneumatische Antriebseinheit, bestehend aus einem Arbeitszylinder 26 mit Antriebskolben vorgesehen. Der Antrieb kann aber auch über einen elektrischen Antrieb 27, z. B. einen Schrittmotor oder Linearmotor erfolgen. Die Federn 22 dienen der Rückstellung des Balg 23. Der Druck im Balg entspricht etwa dem peep-Niveau, wird jedoch zweckmäßigerweise ca. 5 mbar höher eingestellt.

Die Entleerung des Balgs erfolgt über eine Leitung 2ß und einem regelbaren Ventil 25.

Der exspiratorische Zweig 13 besteht aus einem exspiratorischen Kanal 30 an dessen Ende sich ein Exspirationsventil 31 anschließt, welches als ansich bekanntes Peep-Ventil ausgebildet sein kann.

Das Exspirationsventil 31 hat lediglich die Funktion auf/zu. Um den kontinuierlich positiven Atemwegdruck (CPAP) zu erhalten muß es jedoch bis zu einem bestimmten Druckniveau (Peep-Niveau) schrießen. Dies läßt sich auf einfache Weise dadurch erreichen, daß der inspiratorische Kanal 16 über eine Druckleitung 32 mit einer Pneumatikeinrichtung 33 des Exspirationsventils 31 verbunden ist. Der Druck in der Leitung 16 (Peep-Niveau) beaufschlagt einen Kolben 34, dessen Kolbenstange auf einen Dichtungsteller 35 einwirkt, der ähnlich einer Ventilabdichtung, den Ausgang des exspiratorischen Kanals 30 abdichtet. Das Exspirationsventil 31 weist weiterhin einen Atemgasaustritt 3. auf. Der Exspirationskanal 30 weist eine. Reihe von Meßeinrichtungen auf. Dies sind insbesondere ein Drucksensor 37 mit Messverstärker 38 der über eine Leitung 35 mit dem Magnetventil 25 der Druckkonstanthaltereinrichtung 21 verbunden ist. Weiterhin sind eine Druckanzeige 40 sowie ein Strömungsmesser 41 jeweils mit Alarmanzeige vorgesehen.

Ein dem Peep-Ventil 18 zusätzlich nachgeschaltetes regelbares Auf-/Zu-Ventil 42 ermöglicht die schon beschriebene Erweiterung des Gerätes als Beatmungsgerät (Flow-Zerhacker). Ein Sicherheitsventil 43 im Inspirationszwelg verhindert einen event. Überdruck bei fehlerhafter Regelung des Ventils 42 bzw. sonstigen Aggregaten.

Die prinzipielle Funktionsweise eines CPAP-Atemhilfegeräts wurde schon eingangs ausführlichst geschildert. Nachstehend seien noch die Unterschiede des Ausführungsbeispiels zum Stand der Technik erläutert:

Während der Inspiration des Patienten 11 gelangt der am Rotameter 17 eingestellte Gasstrom unmittelbar über die Leitung 16 zum Patienten, da im Inspirationszweig 12 ein Sog entsteht und das Exspirationsventil 31 geschlossen ist. Reicht der eingestellte Gasstrom nicht aus, dann sinkt der Gesamtdruck im System solange ab, bis der durch den Drucksensor 37 festgestellte Druck eine eingestellte Unterdruckgrenze erreicht und dadurch vom Meßverstärker 31 ein Signal an das Magnetventil 25 geleitet wird. Durch Öffnen des Ventils 25 gelangt ein zusätzlichss Gasvolumen aus dem Druckkonstanthalter 21 in den Inspirationskanal 1. und damit zum patienten.

Durch die Anordnung des Peep-Ventils 19 im Inspirationszweig 12, gelangt nur das unmittelbar benötigte Gas zum Patienten 11. Der Rest strömt über das Peep-Ventil (überströmventil) 18 ins Freie ab, wodurch gleichzeitig das eingestellte Druckniveau (Peep-Niveau) erreicht und das Magnetventil 25 wieder geschlossen wird.

Bei der darauffolgenden Exspiration erzeugt der Patient 11 einen vom Strömungswiderstand und der Exspirations-Gasgeschwindigkeit abhängigen Überdruck im System, wodurch das Einwegventil 15 geschlossen und das Exspirationsventil 31 geöffnet wird. Die exspirierte Luft gelangt dann über die Messeinrichtungen 40, 41 zum Exspirationsventil 31 und von dort über den Ausgang 36 in die Umgebung. Durch die im Exspirationsventil 31 angeordnete Pneumatikeinrichtung 33 - 35, muß der im Ex-Spirationskanal 30 herrschende Druck größer sein als das eingestellte Peep-Niveau, damit die Exspirationsluft den Ausgang 3. des Exspirationsventils 31 verlassen kann. Der Patient steht deshalb ständig unter dem durch das Peep-Niveau eingestellten positiven Atemwegdruck . (CPAP).

Während der Exspirationsphase wird das System des Druckkonstanthalters 21 wieder aufgefüllt und der am Rotameter 17 eingestellte Gasstrom über das Peep-Ventil abgeleitet. Am Ende der Exspiration sinkt der vom Patienten im System erzeugte Überdruck langsam ab, bis das am Ventil 18 eingestellte Druckniveau das Exspirationsventil 31 wieder schließt.

Die Schwankungsbreite des Atemwegdrucks um das Peep-Niveau, welches ca. 5 - 10 mbar beträgt, d. h. die Unter- und Überschwingweiten um das eingestellte Druckniveau betragen mit der erfindungsgemäßen Einrichtung maximal 3 mbar. Diese Wirkung ergibt sich durch das weiche Ankoppeln des Geräts an den Patienten und einem eingestellten Low-Flow, d. h. einer konstanten minimalen Gasmenge, sowie einer schnellen Ansprechzeit und Gaszuführung der Druckkonstanthalteeinrichtung 21. Dabei ist es von besonderer Bedeutung, daß das peep-Ventil nicht mehr am Ende des Exspirationszweigs, sondern im Inspirationszweig angeordnet ist, so daß der Exspirationszweig ausschließlich ausgeatmete Luft vom patienten aufnehmen muß. Dies ermöglicht niedrigere Atemweg-Druckverhältnisse bzw. Druckschwankungen und genaue Messung des Atemvolumens.

## Patentansprüche

1. Atemhilfegerät zur Wiederherstellung der Spontanatmung von Patienten, mit einem inspiratorischen- und einem exspiratorischen Zweig (12, 16; 13), wobei ein kontinuierlich positiver Atemwegdruck (CPAP) auf Peep-Niveau einstellbar ist, wobei im inspiratorischen Zweig Mittel (15) zum Erzeugen eines dauernd fließenden Gasstroms vorgesehen sind, dessen Druck durch ein Druckbegrenzungsventil (17) bestimmbar ist, und wobei der exspiratorische Zweig durch ein Exspirationsventil (31) schließbar ist, welches auf einen Gasdruck bei Peep-Niveau anspricht, dadurch gekennzeichnet, daß im inspiratorischen Zweig ein Peep-Ventil (18) zur Einstellung des Peep-Niveaus vorgesehen ist, daß das Exspirationsventil (31) und damit der Druck im Exspirationszweig (13) durch eine den Druck im inspiratorischen und exspiratorischen Zweig vergleichende Regeleinrichtung (32 bis 35) auf Peep-Niveau steuerbar ist und daß mittels eines Einwegventils (15) im inspiratorischen Zweig (12, 16) dieser in der exspiratorischen Phase vom Patienten (11) abgetrennt ist, wobei der dauernd fließende Gasstrom aus dem inspiratorischen Zweig (12) in der exspiratorischen Phase über das Peep-Ventil (18) ableitbar ist.

2. Atemhilfegerät nach Anspruch 1, dadurch gekennzeichnet, daß der inspiratorische (12, 16) vom exspiratorischen Zweig (13, 30) durch ein Schlauchsystem mit Patienten-Anschluß-T-Stück (14) getrennt ist.

3. Atemhilfegerät nach Anspruch 1, dadurch gekennzeichnet, daß das Einwegventil (15) als Glimmerventil ausgebildet ist.

4. Atemhilfegerät nach Anspruch 1, dadurch gekennzeichnet, daß das Peep-Ventil (16) im inspiratorischen Zweig als Standrohr mit Wasserschloß ausgebildet und auf ein Peep-Niveau von 5 bis 25 mbar, insbesondere bis 10 mbar einstellbar sind.

5. Atemhilfegerät nach Anspruch 1, dadurch gekennzeichnet, daß das Exspirationsventil (31) als pneumatisches Druckventil (33) ausgebildet ist, wobei der exspiratorische Zweig (13, 30) durch den Druck im inspiratorischen Zweig (12, 6) (Peep-Niveau) mittels einer Schließeinrichtung (32 bis 35) verschlossen ist.

6. Atemhilfegerät nach Anspruch 1, dadurch gekennzeichnet, daß der kontinuierlich positive Atemwegdruck (CPAP) durch einen Hochdruckgasmischer (15) erzeugbar ist, wobei eine Druckminderung mittels eines Durchflußmessers (17) mit Druckminderer erfolgt.

7. Atemhilfegerät nach Anspruch 1, dadurch gekennzeichnet, daß der exspiratorische Zweig (13, 30) Mittel (37, 38, 40, 41) zur Messung des Exspirationsvorganges aufweist.

8. Atemhilfegerät nach einem oder mehreren der vorhergehenden Ansprüche 1 bis 7, dadurch gekennzeichnet, daß dem Peep-Ventil (18) im inspiratorischen Zweig (12) ein über eine Zeit-Takt-Schaltung aussteuerbares Auf- und Zu-Ventil (42) nachgeschaltet ist.

9. Atemhilfegerät nach Anspruch 8, dadurch gekennzeichnet, daß ein auf beliebigen Druck einstellbares, unabhängiges Sicherheitsventil (43) vorgesehen ist.

10. Atemhilfegerät nach Anspruch 1 oder 6, dadurch gekennzeichnet, daß zur zusätzlichen Einspeisung von Gas in einem Low-Flow-CPAP-System dem inspiratorischen Zweig (12, 16) ein über ein Füllstandsregelventil (25) mit einem Hochdruckgasmischer (15) verbundener Druckkonstanthalter (21) parallelgeschaltet ist, dessen gespeichertes Gasvolumen bei Bedarf dem inspiratorischen Zweig (12) zuführbar ist.

11. Atemhilfegerät nach Anspruch 10, dadurch gekennzeichnet, daß die Druckkonstanthalteeinrichtung (21) als federbelasteter Balg (23) ausgebildet ist, dessen Gasvolumen über ein regelbares Magnetventil (25) dem inspiratorischen Zweig (12) zuführbar ist, wobei der Atemwegdruck des Patienten in der inspiratorischen Phase derart als Regelgröße dient, daß die Regelung des Magnetventils (25) über einen vom Peep-Niveau abhängigen Drucksensor (37) erfolgt.

**Claims**

1. Auxiliary respirator to respiration in patients. with an inhalation and an exhalation branch (12, 16; 13), a continuously positive airway pressure (CPAP) being adjustable to peep level. means (15) provided in the inspiratory branch to genarate a constantly flowing etream of gas, the pressure of which can be determined by a pressure limiting valve (17), the expiratory branch being adapted to be closed by an exhalation valve (31) which responds to a gas pressure at peep level characterised in that a peep valve (18) is provided in the inspiratory branch for adjusting the peep level and in that the exhalation or expiratory valve (31) and thus the pressure in the expiretory branch (13) can be controlled to peep level by a regulating device (32 to 35) which compares the pressure in the inspiratory and expiratory branches and in that by means of a one-way valve (19) in the inspiratory branch (12, 16) this latter becomes isolated from the patient (11) during the expiratory of exhalation phase, it being possible for the constantly flowing stream of gas to be diverted through the peep valve (18) out of the inspiratory branch (12) during the expiratory or exhalation phase.

2. Auxiliary respiratory according to Claim 1, characterised in that the inspiratory branch (12, 16) and the isolator from the expiratory branch (13, 30) by a hose system with a patient-connection T-piece (14).

3. Auxiliary respirator according to Claim characterised in that the one-way valve (19) is constructed as a mica valve.

4. Auxiliary respirator according to Claim 1. characterised in that the peep valve (18) in the inspiratory branch is constructed as a standpipe with a water lock and can be adjusted to a peep level of 5 to 25 mbar and in particular 8 to 10 mbar.

5. Auxiliary respirator according to Claim 1, characterised in that the expiratory valve (31) is constructed as a pneumatic pressure valve 33, the expiratory branch (13, 30) being closed by means of a closure device (32 to 25) by virtue of the pressure in the inspiratory branch (12, 6) (peep level).

6. Auxiliary fespirator according to Claim 1, characterised in that the continuously positive air-way pressure (CPAP) can be generated by a high pressure gas blender (18), a reduction in pressure taking place by a through flow meter (17) with a pressure reducer.

7. Auxiliary respirator according to Claim 1, characterised in that the expiratory branch (13, 30) comprises means (37, 38, 40, 41) for measuring the expiration process.

8. Auxiliary respirator according to one or more of the preceding Claims 1 to 7, characterised in that areas in the inspirator branch (12) and downstream of the peep valve (18) and open and close valve (42) which can be operated by a timeclock circuit.

9. Auxiliary respirator according to Claim 8, characterised in that an independent safety valve (43) is provided which can be adjusted to any desired pressure.

10. Auxiliary respirator accordins to Claim 1 or 8, characterised in that for the additional feeding of gas in a low-flow CPAP system areas connected in parallel with the inspirator branch (12, 16) a device (21) for maintaining a constant pressure which is connected via a filling level regulating valve (25) to a high pressure gas blender (15), the volume of gas stored being capable of being fed to the inspiratory branch (12) when required.

11. Auxiliary respirator according to Claim 10, characterised in that the device (21) for maintaining a constant pressure is constructed as a spring-loaded bellows (23) the gas volume of which can be fed through a regularly magnetic valve (29) to the inspiratory branch (12), the air-way pressure of the patient in the inspiratory phase so servcing as a control magnitude that the magnetic valve (29) is regulated via a pressure sensor (37) which is dependent upon the peep level.


**Revendications**

1 - Appareil d'aide respiratoire pour le rétablissement de la respiration spontanée de patients, comportant une branche inspiratoire (12, 16) et une branche expiratoire (13), par quoi une pression de voie respiratoire continuellement positive (CPAP) est réglable à un niveau dit "peep" ou de décharge, des moyens (15) étant prévus dans la branche inspiratoire pour créer un courant gazeux permanent, dont la pression peut être déterminée par l'intermédiaire d'une soupape (17) de limitation de pression, et la branche expiratoire pouvant être fermée par une soupape d'expiration (31), laquelle réagit à une pression de gaz pour le niveau de décharge, caractérisé en ce que, dans la branche inspiratoire, est prevue une soupape dite "peep" ou de decharge (18) pour le réglage du niveau de décharge, en ce que la soupape d'expiration (31) et, ainsi, la pression dans la branche expiratoire (13) peut être commandée au niveau de décharge par un dispositif de réglage (32 à 35) comparant la pression dans la branche inspiratoire et dans la branche expiratoire, et en ce que, au moyen d'une soupape une-voie (19) dans la branche inspiratoire (12, 16), celle-ci est séparée du patient (11) dans la phase expiratoire, par quoi le courant gazeux s'ecoulant de façon permanente peut être détourné de la branche inspiratoire (12) dans la phase expiratoire par l'intermédiaire de la soupape de décharge (18).

2 - Appareil d'aide respiratoire selon la revendication 1, caractérisé en ce que la branche inspiratoire (12, 16) est séparée de la branche expiratoire (13, 30) par l'intermédiaire d'un système de tuyaux avec pièce de liaison au patient en T (14).

3 - Appareil d'aide respiratoire selon la revendication 1, caractérisé en ce que la soupape une-voie (19) est réalisée sous forme d'une soupape à seuil.

4 - Appareil d'aide respiratoire selon la revendication 1, caractérisé en ce que la soupape de décharge (16) dans la branche inspiratoire est realisée sous forme d'un tuyau de montée avec chambre de compensation et est réglable à un niveau de décharge de 5 à 25 mbar, en particulier de 8 à 10 mbar.

5 - Appareil d'aide respiratoire selon la revendication 1, caractérisé en ce que la soupape d'expiration (31) est réalisée sous forme d'une soupape de pression pneumatique (33), la branche expiratoire (13, 30) étant fermée par la pression dans la branche inspiratoire (12, 16) (niveau de décharge) au moyen d'un dispositif de fermeture (32 à 35).

6 - Appareil d'aide respiratoire selon la revendication 1, caractérisé en ce que la pression respiratoire continuellement positive (CPAP) peut être créée par l'intermédiaire d'un mélangeur de gaz à pression élevée (15), une diminution de pression ayant lieu au moyen d'un débitmètre (17) avèc detendeur.

7 - Appareil d'aide respiratoire selon la revendication 1, caractérisé en ce que la branche expiratoire (13, 30) comporte des moyens (37, 36, 40, 41) pour mesurer le processus d'expiration.

8 - Appareil d'aide respiratoire selon une ou plusieurs des revendications précédentes 1 à 7, caractérisé en ce qu'une soupape (42) marche/arrêt commandable par un montage de cycle de temps est intercalée après la soupape de décharge (18) dans la branche inspiratoire (12).

9 - Appareil d'aide respiratoire selon la revendication 8, caractérisé en ce qu'une soupape de sécurité (43) indépendante et réglable à une pression quelconque est prévue.

10 - Appareil d'aide respiratoire selon la revendication 1 ou 6, caractérisé en ce que, pour une alimentation additionnelle en gaz, dans un système "Low-Flow-CPAP", un stabilisateur de pression (21) relié à un mélangeur de gaz à pression élevée (15) par l'intermédiaire d'une soupape de réglage de niveau (25) est monté en parallèle à la branche inspiratoire (12, 16), dont le volume de gaz emmagasiné peut être envoyé en cas de besoin dans la branche inspiratoire (12).

11 - Appareil d'aide respiratoire selon la revendication 10,

caractérisé en ce que le dispositif stabilisateur de pression (21) est réalisé sous forme d'un soufflet (23) sollicité par ressorts, dont le volume de gaz peut être envoyé à la branche inspiratoire (12) par l'intermédiaire d'une soupape magnétique réglable (29), la pression de voie respiratrice du patient dans la phase inspiratoire servant de grandeur de réglage, de sorte que le réglage de la soupape magnétique (29) se produit par l'intermédiaire d'un détecteur de pression (37) dépendant du niveau de décharge.